# EUROPEAN PATENT SPECIFICATION

(11) **EP 3 153 160 B1**
(45) Date of publication and mention of the grant of the patent: **11.08.2021**
(21) Application number: 15188975.5
(22) Date of filing: 08.10.2015
(51) Int. Cl.: A61K 31/122, A61K 45/00, A61P 35/00

(54) **USE OF XANTHOPHYLLS FOR THE TREATMENT OF CANCERS**
VERWENDUNG VON XANTHOPHYLLEN ZUR KREBSBEHANDLUNG
UTILISATION DE XANTHOPHYLLES POUR LE TRAITEMENT DE CANCERS

(43) Date of publication of application: 12.04.2017
(73) Proprietor: Lu, Chuen Wei, 33058 Taoyuan City (TW)
(72) Inventor: Lu, Chuen Wei, 33058 Taoyuan City (TW)
(74) Representative: Grünecker Patent- und Rechtsanwälte PartG mbB

(56) References cited:
- WO-A1-03/041695
- WO-A2-02/05827
- WO-A2-2006/099015
- US-A1- 2007 259 843
- US-A1- 2013 302 319
- None

## Description

The present invention relates to a novel use of xanthophylls for treating cancers, particularly, adrenocortical carcinoma, osteosarcoma or glioblastoma multiforme.

Xanthophylls are a large group of carotenoids containing oxygen in molecules in addition to carbon and hydrogen. Astaxanthin, a xanthophyll carotenoid, is a natural antioxidant that is produced in many biological organisms (Hussein et al., 2006). It occurs naturally in certain algae and causes the pink or red color in salmon, trout, lobster, shrimp, and other seafood. While astaxanthin is a natural dietary component, it can also be used as a food supplement. The U.S. Food and Drug Administration (FDA) has approved astaxanthin as a food coloring (or color additive) for specific uses in animal and fish foods. The European Commission considers it a food dye and it is given the E number E161j. Natural astaxanthin is generally recognized as safe (GRAS) by the FDA, but as a food coloring in the United States it is restricted to use in animal food only.

The biological activities of astaxanthin have been tested in many animal models and clinical tests. Astaxanthin blocks the aerobic metabolism-generated free radicals, such as hydroxyls, peroxides and reactive oxygen species, thus preventing abnormal DNA damage (Higuera-Ciapara et al., 2006). Further studies have also demonstrated that astaxanthin contributes to immune response (Kim et al., 2009; Park et al., 2010). Participants who received astaxanthin daily for 8 weeks showed upregulating mitogen-induced lymphoproliferation, increased natural killer cell cytotoxic activity, and increased total T and B leukocytes. US 6,773,708 B1 discloses the use of xanthophylls such as astaxanthin for the treatment of autoimmune diseases, chronic viral and intracellular bacterial infections by suppressing excessive Th1 cell mediated immune responses, and stimulating Th2 cell mediated immune responses in a patient during ongoing infection and/or inflammation in said patient.

Oral administration of astaxanthin inhibits thrombosis in vascular endothelial injury mouse models and in stroke-prone spontaneously hypertensive rats (Khan et al., 2010; Sasaki et al., 2011). CN 101361726 A discloses that astaxanthin can improve behavioral symptoms caused by cerebral ischemia reperfusion injury of rats, decrease the volume of cerebral infarction and lower the cerebral edema of cerebral ischemic rats, and thus can be used for preparing drugs for preventing and curing brain stroke.

In addition, astaxanthin also protects neuronal cells. Treatment with astaxanthin ameliorates neuroinflammation and early brain injury by activating nuclear factor erythroid-related factor 2 and the antioxidant responsive element (Nrf2-ARE) pathway after subarachnoid hemorrhage (Wu et al., 2014; Zhang et al., 2014a; Zhang et al., 2014b). More importantly, astaxanthin penetrates the natural blood-brain barrier (BBB) and thus can be used in treating brain neuronal diseases (Wu et al., 2014). EP1 867 327 A1 discloses a neurocyte protective agent comprising astaxanthin and/or an ester thereof capable of alleviating mitochondrial dysfunction and oxidative stress in neurocytes. The neurocyte protective agent is effective in protecting against the degeneration of dopaminergic neurons of the substantia nigra and noradrenergic neurons of the locus ceruleus, and it is suggested that the neurocyte protective agent can be used for Parkinson's disease.

Astaxanthin is also suggested as a potential anti-tumor compound. It inhibits hamster oral cancer cell growth by blocking STAT, ERK or PI3K/Akt signaling (Kavitha et al., 2013; Kowshik et al., 2014). It was also reported that the treatment of astaxanthin induces mitochondria-depended apoptosis in hepatocellular carcinoma cells (Song et al., 2011). US 6,277,417 B1 discloses that astaxanthin can inhibit the activity of the enzyme 5α-reductase and thus suggests that it can be used for treating or preventing disorders resulting from the activity of the enzyme, in particular benign prostate hyperplasia and prostate cancer.

The adrenal gland, located superiorly to the kidney, consists of the outer adrenal cortex and the inner medulla parts (Else et al., 2014). The adrenal cortex secretes several steroids such as glucocorticoid and mineralocorticoid to regulate the metabolic homeostasis in the body. Adrenocortical carcinoma is a rare disease, with incidence of 1 to 2 per million people every year. It is an aggressive tumor and is often metastasized to other organs, and the overall survival rate is only 20-35% within 5 years. Tumors occurring in the adrenal cortex may induce aberrant secretion of steroids and show symptoms similar to Cushing's syndrome and virilization (Erickson et al., 2014). Several methods are used in the treatment of adrenocortical carcinoma, e.g., complete surgical excision, radiation therapy and combined chemotherapy (the combination of anti-tumor drugs: cisplatin, doxorubicin and etoposide). To date, there is no single method that can effectively block adrenocortical tumor cell growth. It is desirable to develop a therapeutic agent that can safely and effectively treat adrenocortical carcinoma.

Osteosarcoma is the most common histological form of primary bone cancer. It is most prevalent in children and young adults. Mifamurtide (MEPACT®) is currently approved in Europe for the treatment of osteosarcoma. A combination of high-dose methotrexate with leucovorin rescue, intra-arterial cisplatin, adriamycin, ifosfamide with mesna, BCD (bleomycin, cyclophosphamide, dactinomycin), etoposide, and muramyl tripeptide may also be used for the treatment of osteosarcoma.

Glioblastoma multiforme (GBM) is one of the most severe malignant gliomas. GBM is considered incurable, and is associated with high morbidity and mortality, with a median survival of 15 months following several treatments (Aldape et al., 2015; Alifieris and Trafalis, 2015). Temozolomide (TEMODAR®) is currently approved in Europe and the United States for the treatment of GBM. Temozolomide is an alkylating drug indicated for the treatment of adult patients with newly diagnosed GBM concomitantly with radiation therapy and then as maintenance treatment. Several aggressive combination therapies are currently used in treating GBM, such as maximal-safe surgical resection, radiation therapy and temozolomide treatment (Stupp et al., 2009). Many GBM patients respond poorly to the conventional chemotherapy and radiation therapy. The prognosis falls in a poor survival range of 12-15 months (median 14.6 months).

US 8,632,825 B2 discloses the use of tetrahydrocannabinol (THC) and cannabidiol (CBD) in the manufacture of a medicament for use in the treatment of GBM. US 8,058,243 B2 discloses a method for treating a glioblastoma cancer involving neural precursor cells, early neural progenitor cells, neural stem cells (NSCs) or a combination thereof, the method comprising administering to a subject in need thereof a therapeutically effective amount of a composition comprising ifenprodil.

WO 03/041695 A relates to a method of reducing the activity of a cancer cell which comprises administering a composition comprising lycopene and an additional carotenoid. WO 02/05827 A discloses a compoistion which is useful in the treatment of cancer wherein the composition comprises one or more flavonoids and one or more components selected *inter alia* from carotenoids.

WO 2006/099015 A discloses a method for treating a proliferative disease comprising administering a pharmaceutical composition comprising a carotenoid analog or derivative. US 2007/0259843 A describes the use of a compound for the treatment of an eye disorder wherein the compound is a therapeutic agent linked to a carotenoid.

US 2013/0302319 A discloses a composition comprising zeaxanthin or its derivatives for use in treating a malignant tumor.

Up to now, there is no single compound that can efficiently inhibit GBM tumor growth without other anti-tumor drugs.

It is an objective of the present invention to provide medicaments for preventing or treating cancer selected from the group consisting of adrenal cancer, bone cancer and brain cancer.

The present invention also provides a composition for preventing or treating cancer selected from the group consisting of adrenal cancer, bone cancer and brain cancer, comprising a therapeutically effective amount of astaxanthin and a physiologically acceptable carrier.

The present invention further provides a use of astaxanthin in the manufacture of a medicament for preventing or treating cancer selected from the group consisting of adrenal cancer, bone cancer and brain cancer.

In a preferred embodiment of the invention, the adrenal cancer is selected from the group consisting of adrenocortical adenoma, adrenocortical carcinoma, neuroblastoma and pheochromocytoma.

In a more preferred embodiment of the invention, the adrenal cancer is adrenocortical carcinoma.

In a preferred embodiment of the invention, the bone cancer is selected from the group consisting of osteoma, osteoid osteoma, osteochondroma, osteoblastoma, enchondroma, giant cell tumor of bone, aneurysmal bone cyst, fibrous dysplasia of bone, osteosarcoma, chondrosarcoma, Ewing's sarcoma and fibrosarcoma.

In a more preferred embodiment of the invention, the bone cancer is osteosarcoma.

In a preferred embodiment of the invention, the brain cancer is selected from the group consisting of gliomas, meningiomas, pituitary adenomas and nerve sheath tumors, preferably anaplastic astrocytoma, astrocytoma, central neurocytoma, choroid plexus carcinoma, choroid plexus papilloma, choroid plexus tumor, dysembryoplastic neuroepithelial tumour, ependymal tumor, fibrillary astrocytoma, giant-cell glioblastoma, glioblastoma multiforme (GBM), gliomatosis cerebri, gliosarcoma, hemangiopericytoma, medulloblastoma, medulloepithelioma, meningeal carcinomatosis, neuroblastoma, neurocytoma, oligoastrocytoma, oligodendroglioma, optic nerve sheath meningioma, pediatric ependymoma, pilocytic astrocytoma, pinealoblastoma, pineocytoma, pleomorphic anaplastic neuroblastoma, pleomorphic xanthoastrocytoma, primary central nervous system lymphoma, sphenoid wing meningioma, subependymal giant cell astrocytoma, subependymoma and trilateral retinoblastoma.

In a more preferred embodiment of the invention, the brain cancer is glioblastoma multiforme.

In a preferred embodiment of the invention, the astaxanthin is derived from a natural source, such as a culture of the algae Haematococcus sp..

In a more preferred embodiment of the invention, the algae Haematococcus sp. is *Haemotococcus pluvialis.*

In another preferred embodiment of the invention, the astaxanthin is chemically synthesized.

In a preferred embodiment of the invention, the therapeutically effective amount of astaxanthin is from 0.1 mg to 10 g per day in case of oral administration, or 0.01 mg to 5 g per day in case of parenteral administration.

The composition or medicament of the invention can be used alone or in combination with other treatments. In one preferred embodiment, the composition or medicament is for use without radiation therapy and/or chemotherapy. In another preferred embodiment, the composition or medicament is for use in combination with radiation therapy and/or chemotherapy. In a further preferred embodiment, the composition or medicament is administered simultaneously or separately with one or more therapeutic agents for preventing or treating cancer selected from the group consisting of adrenal cancer, bone cancer and brain cancer or managing the symptoms or complications of adrenal cancer, bone cancer and brain cancer.

In a preferred embodiment of the invention, the cancer has been surgically removed from the subject.

The present invention is described in detail in the following sections. Other characterizations, purposes and advantages of the present invention can be easily found in the detailed descriptions and claims of the invention.

### BRIEF DESCRIPTION OF THE DRAWINGS

Figure 1 shows the bright field microscopy of Y1 cells treated with DMSO (control) or 40 µM astaxanthin for 24 (A) or 48 (B) hours.
Figure 2 shows the growth curve of Y1 cells after astaxanthin treatment (blue line) and DMSO treatment (red line). Cells were counted each time from four independent experiments and the mean±S.D. is shown.
Figure 3 shows the quantification of the population of viable Y1 cells in the presence of DMSO or astaxanthin. ** denotes P<0.01 as compared to control (DMSO treated) group.
Figure 4 shows reduced U87MG cell number after astaxanthin treatment. Cells were counted each time from four independent experiments and the mean±S.D. is shown.
Figures 5A and 5B show that the viability of U87MG cells was reduced upon astaxanthin treatment. The population of viable U87MG cells was quantified in the presence of DMSO or different concentrations of astaxanthin.
Figure 6 shows reduced M059K cell number after astaxanthin treatment. Cells were counted each time from four independent experiments and the mean±S.D. is shown.
Figures 7 shows reduced M059J cell number after astaxanthin treatment. Cells were counted each time from four independent experiments and the mean±S.D. is shown.
Figure 8 shows reduced U2OS cell number after astaxanthin treatment. Cells were counted each time from four independent experiments and the mean±S.D. is shown.

Unless otherwise defined herein, scientific and technical terms used in connection with the present invention shall have the meanings that are commonly understood by those of ordinary skill in the art. The meaning and scope of the terms should be clear; however, in the event of any latent ambiguity, definitions provided herein take precedence over any dictionary or extrinsic definition.

As utilized in accordance with the present disclosure, the following terms, unless otherwise indicated, shall be understood to have the following meanings.

The term "adrenocortical carcinoma (ACC)" as used herein refers to an aggressive cancer originating in the cortex (steroid hormone-producing tissue) of the adrenal gland. Adrenocortical carcinoma may present differently in children and adults. Most tumors in children are functional, and virilization is by far the most common presenting symptom, followed by Cushing's syndrome and precocious puberty. Among adults presenting hormonal syndromes, Cushing's syndrome alone is most common, followed by mixed Cushing's and virilization (glucocorticoid and androgen overproduction). Feminization and Conn syndrome (mineralocorticoid excess) occur in less than 10% of cases. Rarely, pheochromocytoma-like hypersecretion of catecholamines has been reported in adrenocortical cancers. Non-functional tumors (about 40%, authorities vary) usually present with abdominal or flank pain, varicocele and renal vein thrombosis or they may be asymptomatic and detected incidentally.

The term "adrenal cancer" includes, but is not limited to adrenocortical adenoma, adrenocortical carcinoma, neuroblastoma and pheochromocytoma.

The term "bone cancer" includes, but is not limited to osteoma, osteoid osteoma, osteochondroma, osteoblastoma, enchondroma, giant cell tumor of bone, aneurysmal bone cyst, fibrous dysplasia of bone, osteosarcoma, chondrosarcoma, Ewing's sarcoma and fibrosarcoma.

The term "osteosarcoma" refers to a cancerous tumor in a bone. Specifically, it is an aggressive malignant neoplasm that arises from primitive transformed cells of mesenchymal origin (and thus a sarcoma) and that exhibits osteoblastic differentiation and produces malignant osteoid.

The term "brain cancer" includes, but is not limited to, gliomas, meningiomas, pituitary adenomas and nerve sheath tumors, preferably anaplastic astrocytoma, astrocytoma, central neurocytoma, choroid plexus carcinoma, choroid plexus papilloma, choroid plexus tumor, dysembryoplastic neuroepithelial tumour, ependymal tumor, fibrillary astrocytoma, giant-cell glioblastoma, glioblastoma multiforme (GBM), gliomatosis cerebri, gliosarcoma, hemangiopericytoma, medulloblastoma, medulloepithelioma, meningeal carcinomatosis, neuroblastoma, neurocytoma, oligoastrocytoma, oligodendroglioma, optic nerve sheath meningioma, pediatric ependymoma, pilocytic astrocytoma, pinealoblastoma, pineocytoma, pleomorphic anaplastic neuroblastoma, pleomorphic xanthoastrocytoma, primary central nervous system lymphoma, sphenoid wing meningioma, subependymal giant cell astrocytoma, subependymoma and trilateral retinoblastoma.

The term "glioblastoma multiforme (GBM)" refers to the highest grade glioma (grade IV) tumor found in humans. GBM also refers to "glioblastoma" under WHO classification. Glioblastomas arise from normal brain tissue. They may invade and migrate away from the main tumor within the brain; however, glioblastoma will rarely spread elsewhere in the body. There are two subtypes of glioblastoma: *de novo* (new or primary) and secondary. De novo tumors arise quickly and tend to make their presence known abruptly. They are the most common, and a very aggressive form of glioblastoma. De novo tumors account for the majority of glioblastomas in persons aged 55 and older. Secondary glioblastomas, most often found in patients aged 45 and younger, typically start as low-grade or mid-grade astrocytoma which have been genetically programmed to eventually transform into malignant, rapidly growing glioblastomas.

The term "radiation therapy" refers to a type of cancer treatment that uses high-energy radiation to kill cancer cells or shrink tumors. The radiation dose used in radiation therapy is measured in gray (Gy), and varies depending on the type and stage of cancer being treated.

The term "chemotherapy" includes, but is not limited to, the administration of a chemotherapeutic compound such as adriamycin, cisplatin, doxorubicin, etoposide, fluoropyrimidine, irinotecan, oxaliplatin, taxol, topotecan, etc.

The term "xanthophylls" as used herein refers to a large group of carotenoids containing oxygen in molecules, in addition to carbon and hydrogen or derivatives thereof. The carotenoids are produced *de novo* by plants, fungi and some bacteria. In a preferred embodiment of the invention, the xanthophyll is astaxanthin.

Astaxanthin and/or an ester thereof is a carotenoid represented by the following formula: 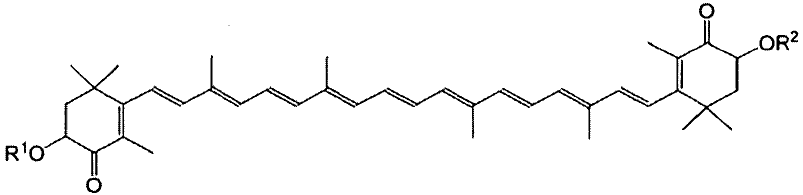 wherein R¹ and R² are both hydrogen in the case of astaxanthin, and R¹ and R² are each independently a hydrogen atom or a fatty acid residue provided that at least one of R¹ and R² is a fatty acid residue in the case of an ester of astaxanthin. Examples of the fatty acid residue in the ester of astaxanthin include, but are not limited to, saturated fatty acids such as palmitic acid and stearic acid or unsaturated fatty acids such as oleic acid, linoleic acid, α-linolenic acid, γ-linolenic acid, bishomo-γ-linolenic acid, arachidonic acid, eicosapentaenoic acid, and docosahexaenoic acid. The astaxanthin ester can be any mono- or di-ester, homogeneous or nonhomogeneous. Astaxanthin has a structure in which an additional oxo group and an additional hydroxy group are present at each end of a β-carotene molecule, so that unlike for β-carotene, the stability of the molecule is low. On the other hand, an ester form (e.g., as obtained in an extract from krill) in which the hydroxy groups at both ends are esterified with an unsaturated fatty acid is more stable.

Astaxanthin used in the present invention may be chemically synthesized or derived from a naturally-occurring product. Examples of the naturally-occurring products in the latter case include red yeast; the shell of crustaceans such as Tigriopus (red water flea) and krills; and microalgae such as green algae, which contain astaxanthin. In the present invention, as long as the properties of astaxanthin can be utilized, any extract containing astaxanthin produced by any method can be used. Generally, extracts from those naturally-occurring products can be used, and the extracts may be crude or purified if necessary. In the present invention, a crude extract or a crushed powder of naturally-occurring products, or a purified product or a chemically synthesized product, if necessary, that contains such astaxanthin can be used either alone or in combination.

The term "preventing" or "prophylaxis" as used herein refers to delaying the onset of symptoms of a susceptible subject, reducing the occurrence of a disorder or condition, or inhibiting the occurrence of the disorder or condition, or arresting the development of the disorder or condition.

The term "treating" or "treatment" as used herein denotes alleviating, relieving, reversing and/or improving a disorder or condition or one or more symptoms thereof, or stopping the symptoms of the disease or condition in a susceptible subject.

The term "subject" as used herein denotes animals, especially mammals. In one preferred embodiment, the term "subject" denotes "humans."

The term "therapeutically effective amount" as used herein refers to the amount of an active ingredient used alone or in combination with other treatments/medicaments for preventing or treating cancer selected from the group consisting of adrenal cancer, bone cancer and brain cancer that show therapeutic efficacy.

The term "physiologically acceptable carrier" refers to solvents, diluents, binders, adhesives, adjuvants, excipients, acceptors, stabilizer, analogues, flavoring agents, sweetening agents, emulsifying agents or preservative agents, which are well known to persons of ordinary skill in the art, for manufacturing pharmaceutical or dietary compositions.

The terms "administering" or "administration" as used herein refer to the methods that may be used to enable delivery of the composition or medicament of the present invention to the desired site of biological action. These methods include, but are not limited to, oral, intraduodenal, nasal, parenteral injection (including intravenous, subcutaneous, intramuscular, intravascular or intradermal), topical and rectal administration. In a preferred embodiment, the composition and medicament described herein are administered orally.

Unless otherwise required by context, singular terms shall include the plural and plural terms shall include the singular.

An objective of the present invention is to provide a medicament for preventing or treating cancer selected from the group consisting of adrenal cancer, bone cancer and brain cancer.

The aim of the present invention is to provide medicaments for preventing or treating cancer selected from the group consisting of adrenal cancer, bone cancer and brain cancer.

In the practice of the invention, a composition comprising a therapeutically effective amount of astaxanthin is administered to a subject, e.g., a human subject, in need of the treatment and/or invention to inhibit the growth of tumor cells.

In the practice of the invention, xanthophylls from any sources can be used, whether natural or synthetic. According to a preferred embodiment of the invention, the xanthophyll is astaxanthin. In a particularly preferred embodiment of the invention, the astaxanthin is derived from a natural source, such as a culture of the algae Haematococcus sp., e.g., *Haemotococcus pluvialis. Haematococcus pluvialis* microalgae is a preferred natural, commercially available source of the astaxanthin used in this invention. In another preferred embodiment of the invention, the astaxanthin is chemically synthesized. Synthetic methods for preparing astaxanthin are known (R. D. G. Cooper et al., J. Chem Soc. Perkins Trans. I, (1975) p. 2195; F. Kienzle, H. Mayer, Helv. Chim. Acta., (1978) Vol. 61, p. 2609) as are methods of isolating astaxanthin from natural sources (Tischer, Z. Physiol. Chem., (1941) Vol. 267 p. 281; Seybold and Goodwin, Nature, (1959) Vol. 184, p. 1714). Thus, astaxanthin can be administered in a pure form as synthesized or isolated from natural sources.

Alternatively, and as a preferred embodiment of the invention, astaxanthin is administered as part of a composition comprising protein, carbohydrate, and fatty acids. When *Haemotococcus pluvialis* algae meal is used as the source of astaxanthin, the composition is preferrably administered as derived from the microalgae, comprising the natural protein, carbohydrate, and fatty acid components of the microalgae. Such microalgae is commercially available (Cyanotech Corporation, Kailua-Kona, Hi.) and generally comprises as major components (by weight) from about 1.5 to 2% astaxanthin, about 15 to 30% protein, about 35 to 40% carbohydrates, about 10 to 25% ash, about 5 to 20% fat, and about 3 to 10% moisture. The composition further comprises minor components including iron, magnesium, calcium, biotin, L-carnitine, folic acid, niacin, pantothenic acid, and vitamins B1, B2, B6, B12, C, and E.

The present invention also provides a composition for preventing or treating cancer selected from the group consisting of adrenal cancer, bone cancer and brain cancer comprising a therapeutically effective amount of astaxanthinand a physiologically acceptable carrier.

The present invention further provides a use of astaxanthinin the manufacture of a medicament for preventing or treating cancer selected from the group consisting of adrenal cancer, bone cancer and brain cancer.

The route of administration of the composition or medicament for preventing or treating cancer selected from the group consisting of adrenal cancer, bone cancer and brain cancer according to the present invention may be either oral or parenteral. The dosage form is selected appropriately according to the route of administration. Examples thereof include parenteral solutions, infusion solutions, powders, granules, tablets, capsules, pills, enteric-coated preparations, troches, liquids for internal use, suspensions, emulsions, syrups, liquids for external use, poultices, nose drops, ear drops, eye drops, inhalants, ointments, lotions, suppositories, and enteral nutrients. These can be used either alone or in combination depending on the condition of a disease. To prepare these dosage forms, auxiliary substances commonly used in the field of pharmaceutical manufacturing technology, such as excipients, binders, antiseptics, antioxidants, disintegrators, lubricants, and flavoring agents, can be used as necessary.

The dose of the astaxanthinfor preventing or treating cancer selected from the group consisting of adrenal cancer, bone cancer and brain cancer according to the present invention varies depending on the purpose of administration or the individual to whom it is to be administered (gender, age, body weight, etc.). In a preferred embodiment of the invention, the therapeutically effective amount of astaxanthinis from 0.1 mg to 10 g, preferably 1 mg to 4 g, more preferably 4 mg to 500 mg, per day in case of oral administration; or 0.01 mg to 5 g, preferably 0.1 mg to 2 g, more preferably 0.1 to 500 mg, per day in case of parenteral administration.

The composition according to the present invention can be used not only as pharmaceuticals as described above, but also as the category of products regulated as "quasi-drugs," cosmetics, functional food products, nutritional supplements, foods and drinks, and other similar products. When used as quasi-drugs or cosmetics, the composition may be used in conjunction with various auxiliary substances commonly used in the field of quasi-drugs or cosmetics, or other technologies, if necessary. Alternatively, when used as functional food products, nutritional supplements, or foods and drinks, the agent may be used in conjunction with additives commonly used for food products, for example, sweeteners, spices, seasonings, antiseptics, preservatives, germicides, and antioxidants, if necessary. The agent may be used in a desired form such as solution, suspension, syrup, granule, cream, paste, or jelly, or may be shaped, if necessary. The ratio of the ingredients contained in these products is not particularly limited, and can be selected appropriately according to the intended purpose, the mode of usage, and the dosage.

The composition or medicament of the invention can be used alone or in combination with other treatments. In one preferred embodiment, the composition or medicament is for use in combination with radiation therapy and/or chemotherapy. In another preferred embodiment, the composition or medicament is administered simultaneously or separately with one or more therapeutic agents for preventing or treating adrenocortical cancer selected from the group consisting of adrenal cancer, bone cancer and brain cancer or managing the symptoms or complications of adrenal cancer, bone cancer and brain cancer.

Having now generally described the invention, the same may be more readily understood through reference to the following examples, which provide exemplary protocols for the production of the pharmaceutical composition of the invention and their use in the enhancement of the treatment of acute stroke. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

### EXAMPLES

### Example 1: Inhibition of Adrenal Tumor Cell Line

### Cell Culture and Drug Treatment

Mouse adrenocortical Y1 cell line (ATCC, ATCC® CCL-79™) was grown in Dulbecco's modified Eagle medium (DMEM)-F12 supplemented with 10% fetal bovine serum at 37°C in a humidified atmosphere at 5% CO₂.

Astaxanthin (Model NO: ATP-9601A; Yuan Yu Bio-Tech (Taiwan), Co., Ltd) was dissolved in dimethyl sulfoxide (DMSO) at concentration of 20 mM as stock solution.

The Y1 cells were incubated with DMSO or 40 µM of astaxanthin for 1, 2 or 3 days before analysis.

### MTT assay

Cells were washed with PBS after the treatment, followed by addition of 1 ml MTT (3-(4,5-cimethylthiazol-2-yl)-2,5-diphenyl tetrazolium bromide) solution (2 mg/ml in PBS) in each well. After incubation for 3 hours at 37°C, 2 ml of DMSO was added and cells were incubated in the dark for 30 minutes. Absorbance was measured at the wavelength of 570 nm (OD570).

### Results

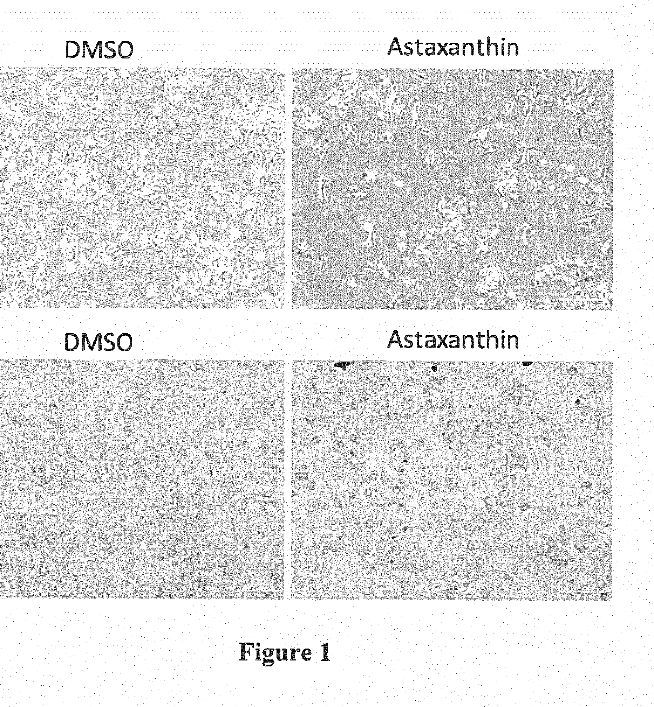

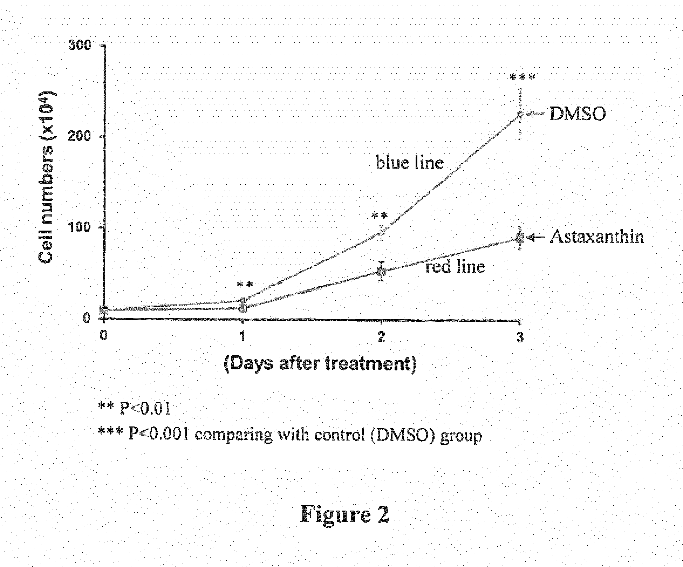

After 1, 2 or 3 days of treatment, the number of Y1 cells in the astaxanthin treated group was reduced as compared with that in the control (DMSO treated) group (see Figures 1 and 2). This result was further confirmed by MTT assay, which measures the activity of mitochondrial succinate dehydrogenase and is indicative of cell viability.

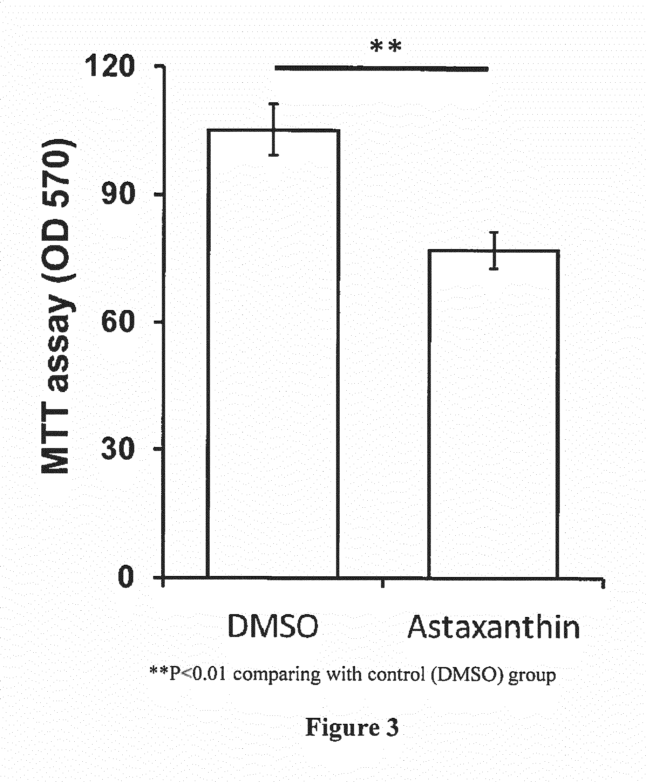

As shown in Fig. 3, after astaxanthin treatment for 48 hours, the OD570 of the treatment group was reduced as compared with that of the DMSO treated group. The data can prove that astaxanthin efficiently inhibits adrenocortical tumor cell growth.

### Example 2: Inhibition of Brain Tumor Cell Lines

### Cell Culture and Drug Treatment

Human U87MG (ATCC, ATCC® HTB-14™) cell line was grown in DMEM medium supplemented with 10% fetal bovine serum at 37°C in a humidified atmosphere at 5% CO₂. M059K (ATCC, ATCC® CRL-2365™) and M059J (ATCC, ATCC® CRL-2366™) cell lines were grown in Dulbecco's modified Eagle medium (DMEM)-F12 supplemented with 10% fetal bovine serum at 37°C in a humidified atmosphere at 5% CO₂.

Astaxanthin was dissolved in dimethyl sulfoxide (DMSO) at concentration of 20 mM as stock solution.

The U87MG, M059K, and M059J cell lines were treated with DMSO or 100, 200 or 400 µM of astaxanthin for 24 hours or 48 hours and cell numbers were counted.

### Results

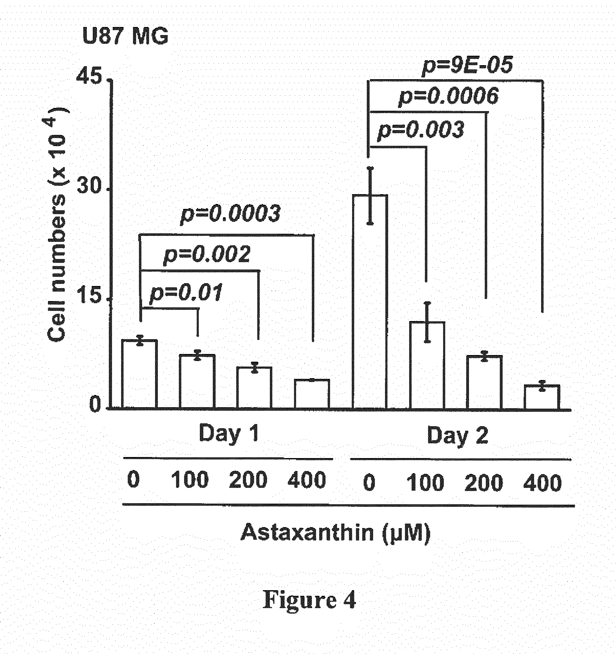

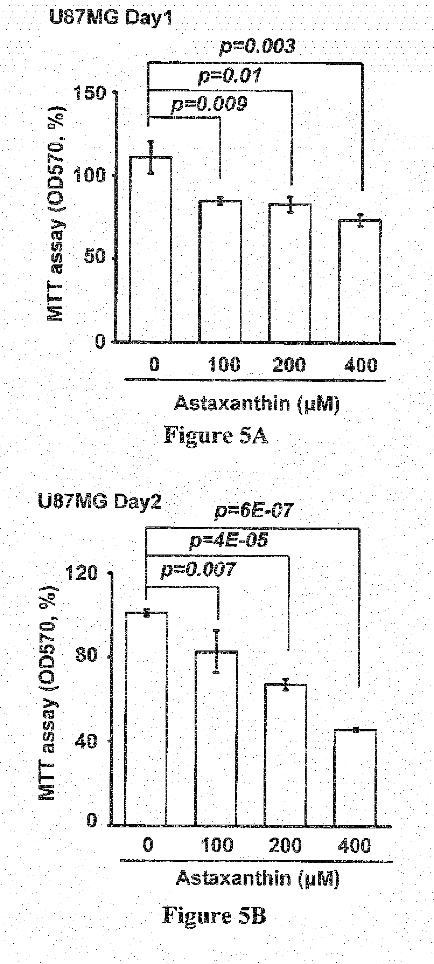

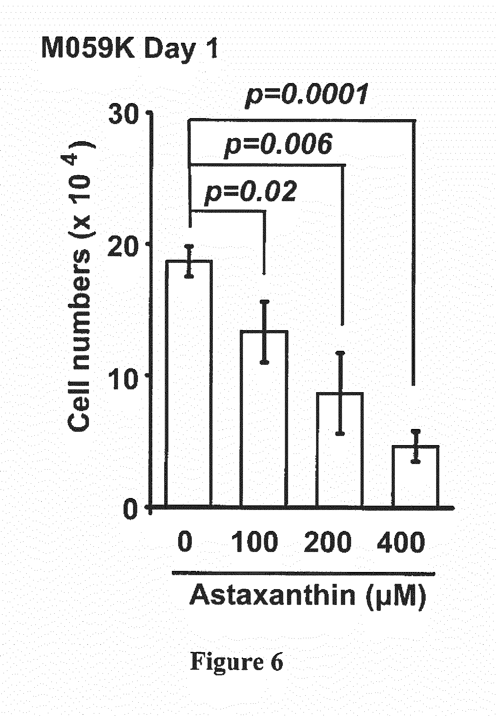

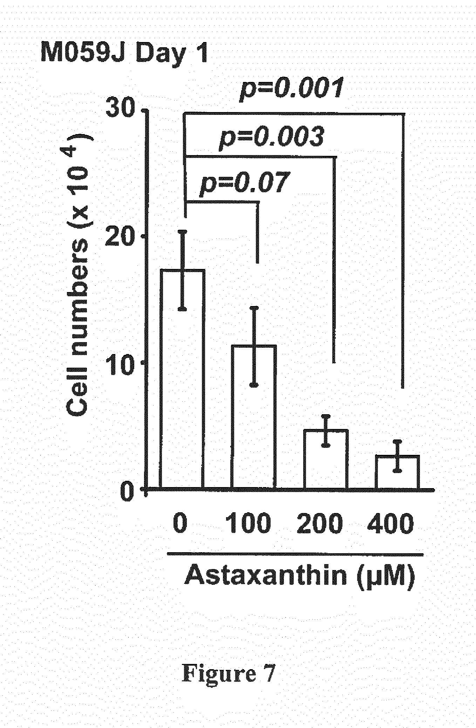

As shown in Figure 4, astaxanthin inhibited U87MG cell growth in a dose-dependent and time-dependent manner. The data were also confirmed by MTT assay (see Figure 5). As shown in Figures 6 and 7, astaxanthin also inhibited M059K (Figure 6) and M059J (Figure 7) cell growth. These data demonstrated that treatment with astaxanthin inhibited glioblastoma cell growth in a dose-dependent manner. Without being limited by theory, it is believed that astaxanthin can penetrate BBB (blood-brain barrier), indicating that astaxanthin might function as an anti-glioblastoma compound.

### Example 3: Inhibition of Bone Tumor Cell Line

### Cell Culture and Drug Treatment

Human U2OS (ATCC, ATCC® HTB-96™) cell line was grown in DMEM medium supplemented with 10% fetal bovine serum at 37°C in a humidified atmosphere at 5% CO₂.

Astaxanthin was dissolved in dimethyl sulfoxide (DMSO) at concentration of 20 mM as stock solution.

The U2OS cell line was treated with DMSO or 100, 200 or 400 µM of astaxanthin for 72 hours and cell numbers were counted.

### Results

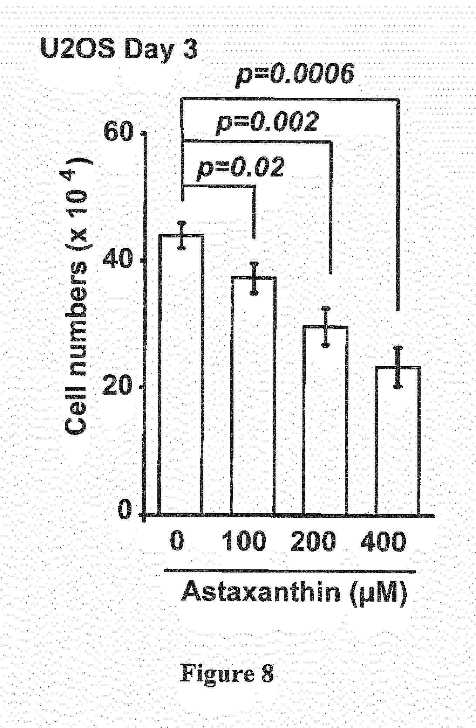

As shown in Figure 8, astaxanthin inhibited U2OS cell growth in a dose-dependent manner. These data demonstrate that treatment with astaxanthin inhibited osteosarcoma cell growth in a dose-dependent manner. The data can prove that astaxanthin efficiently inhibits osteosarcoma cell growth.

These data demonstrate that the xanthophylls (astaxanthin) can efficiently inhibit adrenocortical tumor, bone tumor and glioblastoma growth without any radiation therapy and/or chemotherapy.

Numerous modifications and variations of the invention as set forth in the above illustrative examples are expected to occur to those skilled in the art. Consequently, only such limitations as appear in the appended claims should be placed on the invention.

### References

Aldape, K., G. Zadeh, S. Mansouri, G. Reifenberger, and A. von Deimling. 2015. Glioblastoma: pathology, molecular mechanisms and markers. Acta neuropathologica*.*
Alifieris, C., and D.T. Trafalis. 2015. Glioblastoma multiforme: Pathogenesis and treatment. Pharmacology & therapeutics*.*
Else, T., A.C. Kim, A. Sabolch, V.M. Raymond, A. Kandathil, E.M. Caoili, S. Jolly, B.S. Miller, T.J. Giordano, and G.D. Hammer. 2014. Adrenocortical carcinoma. Endocrine reviews. 35:282-326.
Erickson, L.A., M. Rivera, and J. Zhang. 2014. Adrenocortical carcinoma: review and update. Advances in anatomic pathology. 21:151-159.
Higuera-Ciapara, I., L. Felix-Valenzuela, and F.M. Goycoolea. 2006. Astaxanthin: a review of its chemistry and applications. Critical reviews in food science and nutrition. 46:185-196.
Hussein, G., U. Sankawa, H. Goto, K. Matsumoto, and H. Watanabe. 2006. Astaxanthin, a carotenoid with potential in human health and nutrition. Journal of natural products. 69:443-449.
Kavitha, K., J. Kowshik, T.K. Kishore, A.B. Baba, and S. Nagini. 2013. Astaxanthin inhibits NF-kappaB and Wnt/beta-catenin signaling pathways via inactivation of Erk/MAPK and PI3K/Akt to induce intrinsic apoptosis in a hamster model of oral cancer. Biochimica et biophysica acta. 1830:4433-4444.
Khan, S.K., T. Malinski, R.P. Mason, R. Kubant, R.F. Jacob, K. Fujioka, S.J. Denstaedt, T.J. King, H.L. Jackson, A.D. Hieber, S.F. Lockwood, T.H. Goodin, F.J. Pashkow, and P.F. Bodary. 2010. Novel astaxanthin prodrug (CDX-085) attenuates thrombosis in a mouse model. Thrombosis research. 126:299-305.
Kim, Y.J., Y.A. Kim, and T. Yokozawa. 2009. Protection against oxidative stress, inflammation, and apoptosis of high-glucose-exposed proximal tubular epithelial cells by astaxanthin. Journal of agricultural and food chemistry. 57:8793-8797.
Kowshik, J., A.B. Baba, H. Giri, G. Deepak Reddy, M. Dixit, and S. Nagini. 2014. Astaxanthin inhibits JAK/STAT-3 signaling to abrogate cell proliferation, invasion and angiogenesis in a hamster model of oral cancer. PloS one. 9:e109114.
Park, J.S., J.H. Chyun, Y.K. Kim, L.L. Line, and B.P. Chew. 2010. Astaxanthin decreased oxidative stress and inflammation and enhanced immune response in humans. Nutrition & metabolism. 7:18.
Sasaki, Y, N. Kobara, S. Higashino, J.C. Giddings, and J. Yamamoto. 2011. Astaxanthin inhibits thrombosis in cerebral vessels of stroke-prone spontaneously hypertensive rats. Nutrition research. 31:784-789.
Song, X.D., J.J. Zhang, M.R. Wang, W.B. Liu, X.B. Gu, and C.J. Lv. 2011. Astaxanthin induces mitochondria-mediated apoptosis in rat hepatocellular carcinoma CBRH-7919 cells. Biological & pharmaceutical bulletin. 34:839-844.
Stupp, R., M.E. Hegi, W.P. Mason, M.J. van den Bent, M.J. Taphoorn, R.C. Janzer, S.K. Ludwin, A. Allgeier, B. Fisher, K. Belanger, P. Hau, A.A. Brandes, J. Gijtenbeek, C. Marosi, C.J. Vecht, K. Mokhtari, P. Wesseling, S. Villa, E. Eisenhauer, T. Gorlia, M. Weller, D. Lacombe, J.G. Cairncross, R.O. Mirimanoff, R. European Organisation for, T. Treatment of Cancer Brain, G. Radiation Oncology, and G. National Cancer Institute of Canada Clinical Trials. 2009. Effects of radiotherapy with concomitant and adjuvant temozolomide versus radiotherapy alone on survival in glioblastoma in a randomised phase III study: 5-year analysis of the EORTC-NCIC trial. The Lancet. Oncology. 10:459-466.
Wu, Q., X.S. Zhang, H.D. Wang, X. Zhang, Q. Yu, W. Li, M.L. Zhou, and X.L. Wang. 2014. Astaxanthin activates nuclear factor erythroid-related factor 2 and the antioxidant responsive element (Nrf2-ARE) pathway in the brain after subarachnoid hemorrhage in rats and attenuates early brain injury. Marine drugs. 12:6125-6141.
Zhang, X.S., X. Zhang, Q. Wu, W. Li, C.X. Wang, G.B. Xie, X.M. Zhou, J.X. Shi, and M.L. Zhou. 2014a. Astaxanthin offers neuroprotection and reduces neuroinflammation in experimental subarachnoid hemorrhage. The Journal of surgical research. 192:206-213.
Zhang, X.S., X. Zhang, M.L. Zhou, X.M. Zhou, N. Li, W. Li, Z.X. Cong, Q. Sun, Z. Zhuang, C.X. Wang, and J.X. Shi. 2014b. Amelioration of oxidative stress and protection against early brain injury by astaxanthin after experimental subarachnoid hemorrhage. Journal of neurosurgery. 121:42-54.

## Claims

1. A composition for use in inhibiting tumor cell growth comprising a therapeutically effective amount of astaxanthin wherein the tumor is selected from adrenal cancer, bone cancer and brain cancer.

2. A composition for use in preventing or treating cancer selected from the group consisting of adrenal cancer, bone cancer and brain cancer, comprising a therapeutically effective amount of astaxanthin.

3. The composition for use according to Claim 1 or 2, wherein the adrenal cancer is selected from the group consisting of adrenocortical adenoma, adrenocortical carcinoma, neuroblastoma and pheochromocytoma.

4. The composition for use according to Claim 3, wherein the adrenal cancer is adrenocortical carcinoma.

5. The composition for use according to Claim 1 or 2, wherein the bone cancer is selected from the group consisting of osteoma, osteoid osteoma, osteochondroma, osteoblastoma, enchondroma, giant cell tumor of bone, 1neurismal bone cyst, fibrous dysplasia of bone, osteosarcoma, chondrosarcoma, Ewing's sarcoma and fibrosarcoma.

6. The composition for use according to Claim 5, wherein the bone cancer is osteosarcoma.

7. The composition for use according to Claim 1 or 2, wherein the brain cancer is selected from the group consisting of gliomas, meningiomas, pituitary adenomas and nerve sheath tumors, preferably anaplastic astrocytoma, astrocytoma, central neurocytoma, choroid plexus carcinoma, choroid plexus papilloma, choroid plexus tumor, dysembryoplastic neuroepithelial tumour, ependymal tumor, fibrillary astrocytoma, giant-cell glioblastoma, glioblastoma multiforme (GBM), gliomatosis cerebri, gliosarcoma, hemangiopericytoma, medulloblastoma, medulloepithelioma, meningeal carcinomatosis, neuroblastoma, neurocytoma, oligoastrocytoma, oligodendroglioma, optic nerve sheath meningioma, pediatric ependymoma, pilocytic astrocytoma, pinealoblastoma, pineocytoma, pleomorphic anaplastic neuroblastoma, pleomorphic xanthoastrocytoma, primary central nervous system lymphoma, sphenoid wing meningioma, subependymal giant cell astrocytoma, subependymoma and trilateral retinoblastoma.

8. The composition for use according to Claim 7, wherein the brain cancer is glioblastoma multiforme.

9. The composition for use according to any one of Claims 1-8, wherein the astaxanthin are derived from a natural source and/or are chemically synthesized.

10. The composition for use according to Claim 9, wherein the astaxanthin are derived from a culture of the algae Haematococcus sp..

11. The composition for use according to Claim 10 wherein the algae Haematococcus sp. is *Haemotococcus pluvialis.*

12. The composition for use according to any one of Claims 1-8, where the therapeutically effective amount of astaxanthin is from 0.01 mg to 10 g per day.

13. The composition for use according to any one of Claims 1-8, which is for use alone or in combination with radiation therapy and/or chemotherapy.

14. The composition for use according to any one of Claims 1-8, which is administered simultaneously or separately with one or more therapeutic agents for preventing or treating cancer selected from the group consisting of adrenal cancer, bone cancer and brain cancer, or managing the symptoms or complications of adrenal cancer, bone cancer and brain cancer.

## Patentansprüche

1. Zusammensetzung zur Verwendung bei der Hemmung des Tumorzellwachstums, umfassend eine therapeutisch wirksame Menge an Astaxanthin, wobei der Tumor ausgewählt ist aus Nebennierenkrebs, Knochenkrebs und einer Krebserkrankung des Gehirns.

2. Zusammensetzung zur Verwendung bei der Vorbeugung oder Behandlung von Krebs, ausgewählt aus der Gruppe bestehend aus Nebennierenkrebs, Knochenkrebs und einer Krebserkrankung des Gehirns, umfassend eine therapeutisch wirksame Menge an Astaxanthin.

3. Die Zusammensetzung zur Verwendung nach Anspruch 1 oder 2, wobei der Nebennierenkrebs aus der Gruppe ausgewählt ist, die aus Nebennierenrindenadenom, Nebennierenrindenkarzinom, Neuroblastom und Phäochromozytom besteht.

4. Die Zusammensetzung zur Verwendung nach Anspruch 3, wobei der Nebennierenkrebs ein Nebennierenrindenkarzinom ist.

5. Die Zusammensetzung zur Verwendung nach Anspruch 1 oder 2, wobei der Knochenkrebs ausgewählt ist aus der Gruppe bestehend aus Osteom, Osteoid-Osteom, Osteochondrom, Osteoblastom, Enchondrom, Riesenzelltumor des Knochens, aneurysmatischer Knochenzyste, fibröser Dysplasie des Knochens, Osteosarkom, Chondrosarkom, Ewing-Sarkom und Fibrosarkom.

6. Die Zusammensetzung zur Verwendung nach Anspruch 5, wobei der Knochenkrebs ein Osteosarkom ist.

7. Die Zusammensetzung zur Verwendung nach Anspruch 1 oder 2, wobei die Krebserkrankung des Gehirns ausgewählt ist aus der Gruppe bestehend aus Gliomen, Meningiomen, Hypophysenadenomen und Nervenscheidentumoren, vorzugsweise anaplastisches Astrozytom, Astrozytom, zentrales Neurozytom, Plexus-choroideus-Karzinom, Plexus-choroideus-Papillom, Plexus-choroideus-Tumor, dysembryoplastischer neuroephithelialer Tumor, Ependymom, fibrilläres Astrozytom, Riesenzellglioblastom, Glioblastoma multiforme (GBM), Gliomatosis cerebri, Gliosarkom, Hämangioperizytom, Medulloblastom, Medulloepitheliom, Meningeosis carcinomatosa, Neuroblastom, Neurozytom, Oligoastrozytom, Oligodendrogliom, Meningeom der Sehnervenscheide, pädiatrisches Ependymom, Pilozytisches Astrozytom Pineoblastom, Pineozytom, pleomorphes anaplastisches Neuroblastom, pleomorphes Xanthoastrozytom, primäres Lymphom des Zentralnervensystems, Keilbeinflügel-Meningeom, subependymales Riesenzellastrozytom, Subependymom und trilaterales Retinoblastom.

8. Die Zusammensetzung zur Verwendung nach Anspruch 7, wobei die Krebserkrankung des Gehirns Glioblastoma multiforme ist.

9. Die Zusammensetzung zur Verwendung nach einem der Ansprüche 1-8, wobei das Astaxanthin aus einer natürlichen Quelle stammt und/oder chemisch synthetisiert ist.

10. Die Zusammensetzung zur Verwendung nach Anspruch 9, wobei das Astaxanthin aus einer Kultur der Alge Haematococcus sp. stammt.

11. Die Zusammensetzung zur Verwendung nach Anspruch 10, wobei die Alge Haematococcus sp. *Haemotococcus pluvialis* ist.

12. Die Zusammensetzung zur Verwendung nach einem der Ansprüche 1-8, wobei die therapeutisch wirksame Menge an Astaxanthin von 0,01 mg bis 10 g pro Tag beträgt.

13. Die Zusammensetzung zur Verwendung nach einem der Ansprüche 1-8, die zur Verwendung allein oder in Kombination mit Strahlentherapie und/oder Chemotherapie bestimmt ist.

14. Die Zusammensetzung zur Verwendung nach einem der Ansprüche 1-8, die gleichzeitig oder getrennt mit einem oder mehreren therapeutischen Mitteln zur Vorbeugung oder Behandlung von Krebs, ausgewählt aus der Gruppe bestehend aus Nebennierenkrebs, Knochenkrebs und einer Krebserkrankung des Gehirns, oder zur Behandlung der Symptome oder Komplikationen von Nebennierenkrebs, Knochenkrebs und einer Krebserkrankung des Gehirns verabreicht wird.

## Revendications

1. Composition pour l'utilisation dans l'inhibition de la croissance de cellules tumorales comprenant une quantité thérapeutiquement efficace d'astaxanthine, la tumeur étant sélectionnée parmi le cancer des glandes surrénales, le cancer des os et le cancer du cerveau.

2. Composition pour l'utilisation dans la prévention ou le traitement du cancer sélectionné dans le groupe constitué du cancer des glandes surrénales, du cancer des os et du cancer du cerveau, comprenant une quantité thérapeutiquement efficace d'astaxanthine.

3. Composition pour l'utilisation selon la revendication 1 ou 2, le cancer des glandes surrénales étant sélectionné dans le groupe constitué de l'adénome adrénocortical, du carcinome adrénocortical, du neuroblastome et du phéochromocytome.

4. Composition pour l'utilisation selon la revendication 3, le cancer des glandes surrénales étant le carcinome adrénocortical.

5. Composition pour l'utilisation selon la revendication 1 ou 2, le cancer des os étant sélectionné dans le groupe constitué de l'ostéome, de l'ostéome ostéoïde, de l'ostéochondrome, de l'ostéoblastome, de l'enchondrome, de la tumeur osseuse à cellules géantes, du kyste osseux anévrismal, de la dysplasie fibreuse de l'os, de l'ostéosarcome, du chondrosarcome, du sarcome de Ewing et du fibrosarcome.

6. Composition pour l'utilisation selon la revendication 5, le cancer des os étant l'ostéosarcome.

7. Composition pour l'utilisation selon la revendication 1 ou 2, le cancer du cerveau étant sélectionné dans le groupe constitué des gliomes, des méningiomes, des adénomes de la glande pituitaire et des tumeurs de la gaine des nerfs, préférablement l'astrocytome anaplasique, l'astrocytome, le neurocytome central, le carcinome du plexus choroïdien, le papillome du plexus choroïdien, la tumeur du plexus choroïdien, la tumeur neuroépithéliale dysembryoplasique, la tumeur de l'épendyme, l'astrocytome fibrillaire, le glioblastome à cellules géantes, le glioblastome multiforme (GBM), la *gliomatosis cerebri,* le gliosarcome, l'hémangiopéricytome, le médulloblastome, le médullo-épithélium, la carcinomatose méningée, le neuroblastome, le neurocytome, l'oligoastrocytome, l'oligodendrogliome, le méningiome de la gaine du nerf optique, l'épendymome pédiatrique, l'astrocytome pilocytique, le pinéaloblastome, le pinéocytome, le neuroblastome anaplasique pléomorphe, le xanthoastrocytome pléomorphe, le lymphome du système nerveux central primaire, le méningiome d'une aile du sphénoïde, l'astrocytome sous-épendymaire à cellules géantes, le rétinoblastome sous-épendymaire et trilatéral.

8. Composition pour l'utilisation selon la revendication 7, le cancer du cerveau étant le glioblastome multiforme.

9. Composition pour l'utilisation selon l'une quelconque des revendications 1 à 8, l'astaxanthine étant dérivée d'une source d'origine naturelle et/ou chimiquement synthétisée.

10. Composition pour l'utilisation selon la revendication 9, l'astaxanthine dérivant d'une culture de l'algue *Haematococcus sp.*

11. Composition pour l'utilisation selon la revendication 10, l'algue *Haematococcus sp.* étant *Haemotococcus pluvialis.*

12. Composition pour l'utilisation selon l'une quelconque des revendications 1 à 8, où la quantité thérapeutiquement efficace d'astaxanthine est de 0,01 mg à 10 g par jour.

13. Composition pour l'utilisation selon l'une quelconque des revendications 1 à 8, qui est destinée à l'utilisation seule ou en combinaison avec la radiothérapie et/ou la chimiothérapie.

14. Composition pour l'utilisation selon l'une quelconque des revendications 1 à 8, qui est administrée simultanément ou de manière séparée avec un ou plusieurs agents thérapeutiques pour la prévention ou le traitement du cancer sélectionné dans le groupe constitué du cancer des glandes surrénales, du cancer des os et du cancer du cerveau, ou pour la gestion des symptômes ou des complications du cancer des glandes surrénales, du cancer des os et du cancer du cerveau.
